# EUROPEAN PATENT APPLICATION

(11) **EP 3 238 752 A1**
(43) Date of publication of application: **01.11.2017**
(21) Application number: 15871641.5
(22) Date of filing: 24.06.2015
(51) Int. Cl.: A61L 31/10, A61L 31/14, A61L 31/16

(54) **EXPANSIBLE CARDIOVASCULAR STENT WITH COLLAGEN COVERING MEMBRANE**

(30) Priority: 25.12.2014 CN 201410827012
(71) Applicant: Dongguan Dianfu Product Design Co., Ltd., Dongguan, Guangdong 523000 (CN)
(72) Inventor: LI, Yangde, Dongguan Guangdong 523000 (CN)
(74) Representative: Hryszkiewicz, Danuta
(86) International application number: PCT/CN2015/082156
(87) International publication number: WO 2016/101566

(57) **Abstract**

Disclosed is an expansible cardiovascular stent with a collagen covering membrane, comprising a reticular and tubular stent (1) and a covering membrane attached to the reticular and tubular stent. The reticular and tubular stent (1) and the covering membrane both have a certain extensibility. The covering membrane is laid in the grids of the reticular and tubular stent (1) and the outer side and inner side thereof. The material of the covering membrane is collagen, collagen-chitosan composite material or collagen-calcium phosphate composite material. The reticular and tubular stent (1) is a degradable metal and alloy thereof. The collagen attached to the reticular and tubular stent (1) has a good biocompatibility and compliance, can sufficiently isolate a lesion from blood stream, can be degraded, absorbed and utilized by the body and does not affect the blood vessel endothelialization time.

## Description

The invention relates to the field of cardiovascular stents, and more particularly to an expansible cardiovascular stent comprising a collagen membrane.

With the development of science and technology, the cardiovascular stents are constantly upgraded from the bare stents without medicines to the stents with medicine coatings, and to the biodegradable stents, with increasingly good therapeutic effect and increasingly high safety.

Based on the surface treatment results, the cardiovascular stents are classified into bare stents, coating type stents, and membrane type stents. The bard stents are processed merely by polishing, the coating type stents include surface coating substances such as heparin, titanium oxide, or the like; and the membrane type stents include a metal stent and a layer of degradable or undegradable polymer membrane covering the outer surface of the metal stent.

The membrane type stents maintain the support function of conventional stents, and can effectively prevent abnormal blood flow in diseased vessels, so they are widely used in the treatment of vascular diseases. However, the implantation of membrane type stents increases the probability of restenosis complication. The probability and degree of restenosis complication are closely related to the selection and endothelialization of the membrane, so raw materials having good compliance, capability of separating lesions and blood flow, and good biocompability are preferable. There are many materials that can be used for preparation of membrane stents, for example, polytetrafluorethylene (PTFE) or expanded polytetrafluorethylene (ePTFE), which has high biocompability, good competitive expansibility, small product size, and thus is widely used in clinic. However, in spite of solving abnormal hemodynamics to some extent, the membrane increases the contact area of the stents and blood vessel, prolongs the time of the endothelialization of the blood vessel, so that the stent as a foreign matter is long-term exposed to the blood, thus increasing the probability of thrombogenesis.

In view of the above-described problems, it is one objective of the invention to provide an expansible cardiovascular stent comprising a collagen membrane. The stent is covered by collagen. Collagen is the main component of connective tissues comprising skin, blood vessels, bones, teeth, and cartilage, has good biocompatibility and compliance, can fully separate the lesions and blood flow, can be degraded, absorbed, and utilized by human body, has no influence on the endothelialization time of the blood vessel, so it has huge significance.

To achieve the above objective, in accordance with one embodiment of the invention, there is provided an expansible cardiovascular stent comprising a collagen membrane, comprising: a mesh stent comprising grids formed by tubular structures, an outer surface, and an inner surface; and a membrane coating on the mesh stent; the mesh stent and the membrane are extensible; the membrane is adapted for coating the grids, the outer surface, and the inner surface of the mesh stent.

In a class of this embodiment, the cardiovascular stent further comprises a balloon disposed in an inner cavity of the mesh stent, and the balloon is inflatable to expand the mesh stent, and is shrinkable to withdraw from the mesh stent.

In a class of this embodiment, the mesh stent is a degradable metal selected from the group consisting of magnesium, zinc, calcium, and iron, or an alloy thereof.

In a class of this embodiment, the membrane is collagen, a collagen-chitosan composite or a collagen-calcium phosphate composite.

In a class of this embodiment, the membrane is a collagen-chitosan composite synthesized by electrospinning, and comprises by mass percentage: 3-11% of collagen, 1.5-3% of chitosan, 0.8-1% of polyethylene oxide (PEO), 0-20% of sodium chloride.

In a class of this embodiment, the membrane is a collagen-calcium phosphate composite, a mass percentage of the collagen is 30-40%, and a mass percentage of the calcium phosphate composite is 60-70%.

The invention also provides a method for preparing the stent, the method comprising:
1) preparing the mesh stent;
2) immersing the mesh stent into a collagen solution, and allowing the collagen to attach to the mesh stent; or preparing a collagen-chitosan composite membrane using electrospinning, and coating the mesh stent using the membrane; or immersing the mesh stent into a solution comprising collagen and calcium phosphate, and allowing the collagen to attach to the mesh stent; and
3) freeze drying the stent.

Collagen exists in human skin, skeleton, tooth, tendon, and so on, and is of great importance for the formation of human skin, skeleton, tooth, and cartilage. Collagen is the main component of the connective tissues, accounts for 70 wt. % of skin, 80 wt. % of normal skeleton (in the skeleton, the function of collagen is by means of its reticular formation to bind calcium, phosphorus, minerals and other components to form bone), and almost 100 wt. % of cartilage. Collagen has good biocompatibility with human body. It can be degraded, absorbed and utilized by organism, and will not cause after effect.

The collagen attached to the cardiovascular stent can fully separate the lesions and blood flow, can coagulate and aggregate with blood platelet to prompt hemostasis. The three-screw structure of collagen can induce the attachment of blood platelet, generate the release factor, activate blood coagulation factors to adhere to the bleeding wound, thus blocking the damaged blood vessel.

The expansible cardiovascular stent of the present disclosure has good biocompatibility and compliance, can fully separate the lesions and blood flow, can be degraded, absorbed, and utilized by human body, has no influence on the endothelialization time of the blood vessel, with huge significance.
FIG. 1 is a schematic diagram of an expansible cardiovascular stent comprising a collagen membrane in accordance with one embodiment of the invention;
FIG. 2 is a schematic diagram of an expansible cardiovascular stent comprising a collagen membrane after being expanded in accordance with one embodiment of the invention; and
FIG. 3 is a schematic diagram of grids of an expansible cardiovascular stent comprising a collagen membrane in accordance with one embodiment of the invention.

For further illustrating the invention, experiments detailing an expansible cardiovascular stent comprising a collagen membrane are described hereinbelow combined with the drawings. It should be noted that the following examples are intended to describe and not to limit the invention.

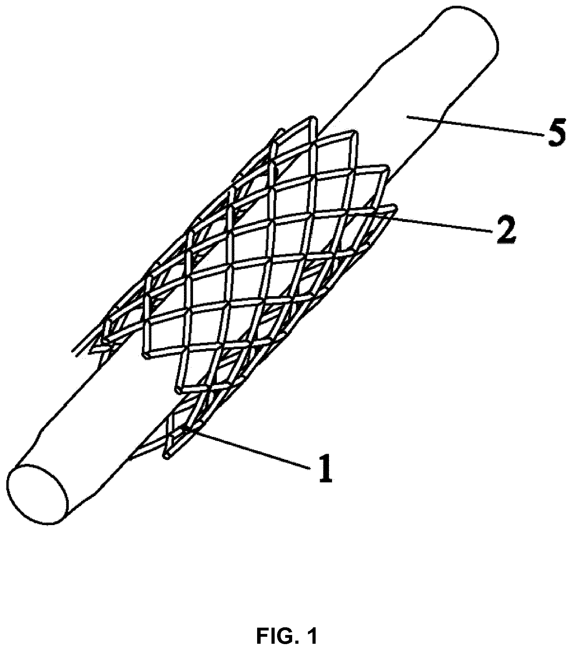

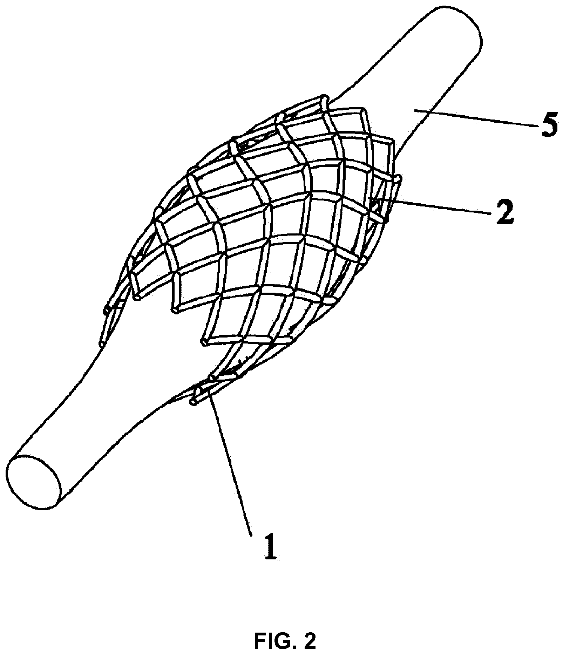

As shown in FIGS. 1 and 2, an expansible cardiovascular stent comprising a collagen membrane comprise a mesh stent 1 and a membrane (not shown in the drawings) coating on the mesh stent. Both the mesh stent and the membrane have elongation, and the membrane is adapted for coating the grids, the outer surface, and the inner surface of the mesh stent 1. The grids of the mesh stent are corrugated or ribbed, or other shapes. It should be noted that, the present disclosure has no limitation to the shape of the grids 2, which is variable as needed.

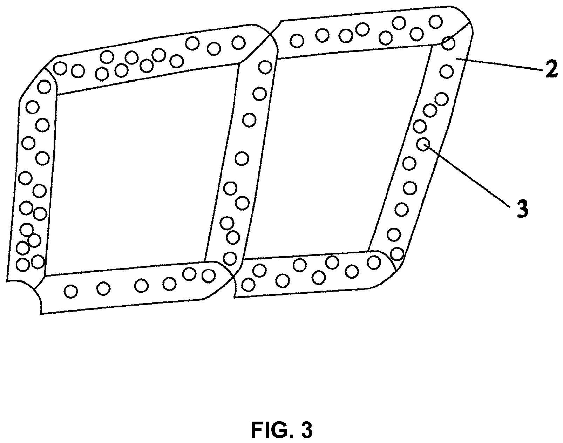

As shown in FIG. 3, the grids 2 of the mesh stent 1 comprise holes 3, which are favorable to the grids 2 to carry medicines. Or, the surface of the grids 2 is uneven, increasing the adhesive force between the grids 2 and the membrane.

The inner cavity of the mesh stent 1 comprises a balloon 5. As shown in FIG. 2, the balloon 5 is inflatable to expand the mesh stent, and is shrinkable to withdraw from the mesh stent.

The mesh stent 1 is a degradable metal selected from the group consisting of magnesium, zinc, calcium, and iron, or an alloy thereof.

The membrane is a collagen-chitosan composite synthesized by electrospinning, and comprises by mass percentage: 3-11% of collagen, 1.5-3% of chitosan, 0.8-1% of polyethylene oxide (PEO), 0-20% of sodium chloride. The membrane is a collagen-calcium phosphate composite, a mass percentage of the collagen is 30-40%, and a mass percentage of the calcium phosphate composite is 60-70%. Collagen has good biocompatibility with human body. It can be degraded, absorbed and utilized by organism, and will not cause after effect.

The collagen attached to the cardiovascular stent can fully separate the lesions and blood flow, can coagulate and aggregate with blood platelet to prompt hemostasis. The three-screw structure of collagen can induce the attachment of blood platelet, generate the release factor, activate blood coagulation factors to adhere to the bleeding wound, thus blocking the damaged blood vessel.

A method for preparing the stent comprises the following steps:
1) preparing the mesh stent;
2) immersing the mesh stent into a collagen solution, and allowing the collagen to attach to the mesh stent; or preparing a collagen-chitosan composite membrane using electrospinning, and coating the mesh stent using the membrane; or immersing the mesh stent into a solution comprising collagen and calcium phosphate, and allowing the collagen to attach to the mesh stent; and
3) freeze drying the stent.

While particular embodiments of the invention have been shown and described, it will be obvious to those skilled in the art that changes and modifications may be made without departing from the invention in its broader aspects, and therefore, the aim in the appended claims is to cover all such changes and modifications as fall within the true spirit and scope of the invention.

## Claims

1. An expansible cardiovascular stent comprising a collagen membrane, comprising:
a mesh stent comprising grids formed by tubular structures, an outer surface, and an inner surface; and
a membrane coating on the mesh stent;
**characterized in that**
the mesh stent and the membrane are extensible;
the membrane is adapted for coating the grids, the outer surface, and the inner surface of the mesh stent.

2. The stent of claim 1, further comprising a balloon disposed in an inner cavity of the mesh stent, wherein the balloon is inflatable to expand the mesh stent, and is shrinkable to withdraw from the mesh stent.

3. The stent of claim 1, **characterized in that** the mesh stent is a degradable metal selected from the group consisting of magnesium, zinc, calcium, and iron, or an alloy thereof.

4. The stent of claim 1, **characterized in that** the membrane is collagen, a collagen-chitosan composite or a collagen-calcium phosphate composite.

5. The stent of claim 4, **characterized in that** the membrane is a collagen-chitosan composite synthesized by electrospinning, and comprises by mass percentage: 3-11% of collagen, 1.5-3% of chitosan, 0.8-1% of polyethylene oxide (PEO), 0-20% of sodium chloride, and a total mass of the collagen and chitosan is four times that of PEO.

6. The stent of claim 4, **characterized in that** the membrane is a collagen-calcium phosphate composite, a mass percentage of the collagen is 30-40%, and a mass percentage of the calcium phosphate composite is 60-70%.

7. A method for preparing the stent of any of claims 1-6, **characterized by** comprising:
1) preparing the mesh stent;
2) immersing the mesh stent into a collagen solution, and allowing the collagen to attach to the mesh stent; or preparing a collagen-chitosan composite membrane using electrospinning, and coating the mesh stent using the membrane; or immersing the mesh stent into a solution comprising collagen and calcium phosphate, and allowing the collagen to attach to the mesh stent; and
3) freeze drying the stent.
